# EUROPEAN PATENT APPLICATION

(11) **EP 1 872 780 A2**
(43) Date of publication of application: **02.01.2008**
(21) Application number: 07110168.7
(22) Date of filing: 13.06.2007
(51) Int. Cl.: A61K 31/167, A61P 25/28, A61P 33/06, A61P 31/18, A61P 43/00

(54) **Omega-Phenyloctanamides as therapeutic compounds**

(30) Priority: 14.06.2006 CH 9712006
(71) Applicant: Speedel Experimenta AG, 4123 Allschwil (CH)
(72) Inventor: Herold, Peter, 4123, Allschwil (CH); Mah, Robert, 4123, Allschwil (CH); Stutz, Stefan, 4123, Allschwil (CH); Tschinke, Vincenzo, 4123, Allschwil (CH); Marti, Christiane, 4123, Allschwil (CH); Schumacher, Christoph, 4123, Allschwil (CH)
(74) Representative: Maué, Paul Georg

(57) **Abstract**

Use of compounds of the general formula (I) 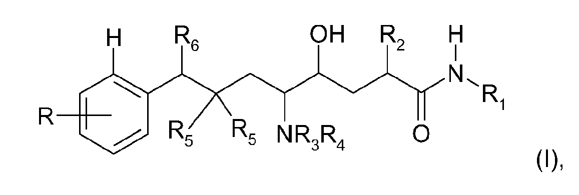 and pharmaceutically usable salts thereof, in which the substituents R, R₁, R₂, R₃, R₄, R₅ and R₆ have the definitions illustrated in detail in the description, as beta-secretase, cathepsin D, plasmepsin II and/or HIV protease inhibitors.

## Description

### Field of the Invention

The present invention relates to the use of w-phenyloctanamides as beta-secretase-, cathepsin D-, plasmepsin II- and/or HIV-protease-inhibitors.

### Background of the Invention

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound Carboxy-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta peptide in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the progression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/ pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histo-aspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, non-peptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity. As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data existing, detailing both the catalytic mechanism and the molecular basis for substrate selection.

### Detailed Description of the Invention

Firstly, the present invention relates to the use as beta-secretase-, cathepsin D-, plasmepsin II- and/or HIV-protease-inhibitors of compounds of the general formula (I): 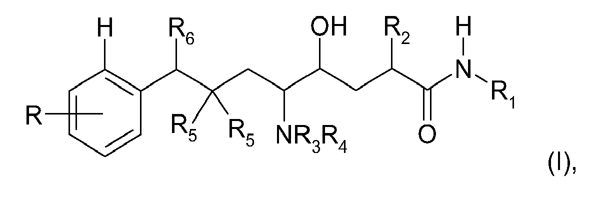 in which
R₁ is unsubstituted or substituted aryl;
R₂ is C₁-C₈-alkyl, C₂-C₈-alkenyl or C₃-C₈-cycloalkyl or is phenyl- or naphthyl-C₁-C₄-alkyl, each of which is unsubstituted or mono-, di- or trisubstituted by C₁-C₄-alkyl, C₁-C₄-alkoxy, hydroxyl, C₁-C₄-alkylamino, N,N-di-C₁-C₄-alkylamino, halogen and/or trifluoromethyl;
R₃ is hydrogen, C₁-C₄-alkyl or C₁-C₈-alkanoyl;
R₄ is hydrogen, C₁-C₄-alkyl or C₁-C₈-alkanoyl;
R₅ are each independently hydrogen, C₁-C₈-alkyl or, together with the carbon atom to which they are bonded, a C₃-C₈-cycloalkylidene radical;
R₆ is hydrogen or hydroxyl;
R are each independently 1-4 radicals selected from:
hydrogen, halogen, C₁-C₈-alkyl, 3- to 8-membered cycloalkyl, polyhalo-C₁-C₄-alkyl, polyhalo- C₁-C₄-alkoxy, C₁-C₄-alkoxy-C₁-C₄-alkyl, C₁-C₄-alkoxy-C₁-C₄-alkoxy-C₁-C₄-alkyl, 3- to 8-membered cycloalkoxy-C₁-C₄-alkyl, hydroxyl, C₁-C₈-alkanoyloxy-C₁-C₄-alkyl, hydroxy-C₂-C₈-alkyl, C₁-C₄-alkylthio-C₁-C₄-alkyl, C₁-C₈-alkanesulphonyl-C₁-C₄-alkyl, thiazolylthio-C₁-C₄-alkyl, thiazolinylthio-C₁-C₄-alkyl, imidazolylthio-C₁-C₄-alkyl, optionally N-oxidized pyridylthio-C₁-C₄-alkyl, pyrimidinylthio-C₁-C₄-alkyl, optionally partially hydrogenated pyridyl- or N-oxidopyridyl-C₁-C₄-alkyl, C₁-C₄-alkanesulphonyl-amino-C₁-C₄-alkyl, trifluoro-C₁-C₈-alkanesulphonylamino-C₁-C₄-alkyl, pyrrolidino-C₁-C₄-alkyl, piperidino-C₁-C₄-alkyl, piperazino-C₁-C₄-alkyl, N'-C₁-C₄-alkylpiperazino-C₁-C₄-alkyl, N'-C₂-C₈-alkanoylpiperazino-C₁-C₄-alkyl, morpholino-C₁-C₄-alkyl, thiomorpholino-C₁-C₄-alkyl, S-oxothiomorpholino-C₁-C₄-alkyl, S,S-dioxothiomorpholino-C₁-C₄-alkyl, cyano-C₁-C₄-alkyl, carboxy-C₁-C₄-alkyl, C₁-C₄-alkoxycarbonyl-C₁-C₄-alkyl, carbamoyl-C₁-C₈-alkyl, N-mono- or N,N-di-C₁-C₄-alkylcarbamoyl-C₁-C₄-alkyl, unsubstituted phenyl or by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted phenyl, unsubstituted naphthyl or by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted naphthyl, hydroxy-C₂-C₈-alkoxy, halo-C₂-C₈-(hydroxy)alkoxy, C₁-C₈-alkanesulphonyl-C₁-C₄-(hydroxy)alkoxy, amino-C₁-C₄-alkyl, C₁-C₄-alkylamino-C₁-C₄-alkyl, N,N-di-C₁-C₄-alkylamino-C₁-C₄-alkyl, N-C₁-C₄-alkanoylamino-C₁-C₄-alkyl, C₁-C₈-alkoxycarbonylamino-C₂-C₈-alkyl, amino-C₁-C₄-alkoxy, C₁-C₄-alkylamino-C₁-C₄-alkoxy, N,N-di-C₁-C₄-alkylamino-C₁-C₄-alkoxy, C₁-C₈-alkanoylamino-C₁-C₄-alkoxy, C₁-C₈-alkoxycarbonylamino-C₂-C₈-alkoxy, C₁-C₈-alkanoyl-C₂-C₄-alkoxy which bears the alkanoyl group in higher than the α-position, C₁-C₈-alkoxy, 3- to 8-membered cycloalkoxy, C₂-C₈-alkenyloxy, 3- to 8-membered cycloalkoxy-C₁-C₄-alkoxy, C₁-C₄-alkoxy-C₁-C₄-alkoxy, C₁-C₄-alkoxy-C₂-C₄-alkenyl, C₂-C₈-alkenyloxy-C₁-C₄-alkoxy, C₁-C₄-alkoxy-C₂-C₄-alkenyloxy, C₂-C₈-alkenyloxy-C₁-C₄-alkyl, C₁-C₄-alkylthio-C₁-C₄-alkoxy, C₁-C₈-alkanesulphonyl-C₁-C₄-alkoxy, C₁-C₄-alkylthio-C₁-C₄-(hydroxy)alkoxy, optionally by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted phenyl-C₁-C₄-alkoxy, optionally by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted naphthyl-C₁-C₄-alkoxy, each of which is unsubstituted or mono-, di- or trisubstituted by C₁-C₄-alkyl, C₁-C₄-alkoxy, hydroxyl, C₁-C₄-alkylamino, di-C₁-C₄-alkylamino, halogen and/or trifluoromethyl, polyhalo-C₁-C₄-alkoxy, optionally partially hydrogenated pyridyl- or N-oxidopyridyl-C₁-C₄-alkoxy, thiazolyl-C₁-C₄-alkoxy, optionally N-oxidized morpholino-C₁-C₄-alkoxy, thiazolylthio-C₁-C₄-alkoxy, thiazolinylthio-C₁-C₄-alkoxy, imidazolylthio-C₁-C₄-alkoxy, optionally N-oxidized pyridylthio-C₁-C₄-alkoxy, pyrimidinylthio-C₁-C₄-alkoxy, C₁-C₈-alkanesulphonylamino-C₁-C₄-alkoxy, trifluoro-C₁-C₈-alkanesulphonyl-C₁-C₄-alkoxy, pyrrolidino-C₁-C₄-alkoxy, piperidino-C₁-C₄-alkoxy, cyano-C₁-C₄-alkoxy, carboxy-C₁-C₄-alkoxy, C₁-C₄-alkoxycarbonyl-C₁-C₄-alkoxy, carbamoyl-C₁-C₄-alkoxy, N-C₁-C₈-alkylcarbamoyl-C₁-C₄-alkoxy, N-mono- or N,N-di-C₁-C₄-alkylcarbamoyl-C₁-C₄-alkoxy, carboxy-C₁-C₄-alkyl, C₁-C₄-alkoxycarbonyl-C₁-C₄-alkyl, carbamoyl-C₁-C₈-alkyl, N-mono- or N,N-di-C₁-C₄-alkylcarbamoyl-C₁-C₄-alkyl, carboxy-C₁-C₄-alkoxy, C₁-C₄-alkoxycarbonyl-C₁-C₄-alkoxy, carbamoyl-C₁-C₈-alkoxy, C₁-C₄-alkylamino or N,N-di-C₁-C₄-alkylamino,
and salts, especially pharmaceutically usable salts thereof.

Aryl contains generally 6-14, preferably 6-10, carbon atoms and is, for example, phenyl, indenyl, e.g. 2- or 4-indenyl, or naphthyl, e.g. 1- or 2-naphthyl. Preference is given to aryl having 6-10 carbon atoms, in particular phenyl or 1- or 2-naphthyl. The radicals mentioned may be unsubstituted or, for example, be mono- or polysubstituted, for example mono- or disubstituted, by C₁-C₈-alkyl, cyano, hydroxyl, amino, C₁-C₆-alkylamino, N,N-di-C₁-C₆-alkylamino, C₂-C₆-alkenyl, C₂-C₆-alkynyl, C₀-C₆-alkylcarbonylamino, C₁-C₆-alkoxycarbonylamino, halogen, trifluoromethyl, C₁-C₈-alkoxy, carbamoyl or N-mono- or N,N-di-C₁-C₈-alkylated carbamoyl, carboxyl or esterified carboxyl, aryl or heterocyclyl, it being possible for the substituent to be in any position, for example in the o-, m- or p-position of the phenyl radical or in the 3-or 4-position of the 1- or 2-naphthyl radical and it also being possible for a plurality of identical or different substituents to be present. Preference is likewise given to aryl having 6-10 carbon atoms, in particular phenyl or 1- or 2-naphthyl with a C₁-C₆-alkylenedioxy radical.

Heterocyclyl contains generally 5 to 7 ring atoms in the heterocyclyl ring which preferably contains one ring nitrogen atom and one further ring heteroatom selected from oxygen, sulphur or nitrogen. Heterocyclyl is, for example, pyridinyl or imidazolyl. Heterocyclyl may be unsubstituted or mono- or polysubstituted, for example mono- or disubstituted, by C₁-C₈-alkyl, halogen, oxide, cyano, amino, C₁-C₆-alkylamino, di-C₁-C₆-alkylamino, oxo, trifluoromethyl, C₁-C₈-alkoxy, carbamoyl or N-mono- or N,N-di-C₁-C₈-alkylated carbamoyl, C₁-C₈-alkoxycarbonyl, aryl or heterocyclyl.

In the case of nitrogen heterocycles, the heterocyclyl radicals can be bonded either via the nitrogen or via a ring carbon.

Halogen is, for example, fluorine, chlorine, bromine or iodine, preferably fluorine and chlorine.

Polyhalo-C₁-C₄-alkyl is, for example, mono-, di-, tri- or tetrahalo-C₁-C₄-alkyl such as fluoromethyl or trifluoromethyl.

Polyhalo-C₁-C₄-alkoxy is, for example, mono-, di-, tri- or tetrahalo-C₁-C₄-alkoxy such as trifluoromethoxy.

3- to 8-membered cycloalkoxy is preferably 3-, 5- or 6-membered cycloalkoxy such as cyclopropyloxy, cyclopentyloxy and cyclohexyloxy.

3- to 8-membered cycloalkyl is preferably 3-, 5- or 6-membered cycloalkyl such as cyclopropyl, cyclopentyl and cyclohexyl.

C₁-C₈-Cycloalkyl-C₁-C₆-alkyl is, for example, cyclopropyl-C₁-C₄-alkyl, cyclobutyl-C₁-C₄-alkyl, cyclopentyl-C₁-C₄-alkyl or cyclohexyl-C₁-C₄-alkyl such as cyclopropylmethyl, cyclobutylmethyl, cyclopentylmethyl, cyclohexylmethyl, cyclopropylethyl, cyclobutylethyl, cyclopentylethyl and cyclohexylethyl

Amino-C₁-C₄-alkoxy is, for example, 2-aminoethoxy, 3-aminopropyloxy or 4-aminobutyloxy.

Amino-C₁-C₄-alkyl is, for example, 2-aminoethyl, 3-aminopropyl or 4-aminobutyl.

Carbamoyl-C₁-C₈-alkyl is, for example, carbamoylmethyl, 2-carbamoylethyl, 3-carbamoylpropyl, 2-(3-carbamoyl)propyl, 2-carbamoylpropyl, 3-(1-carbamoyl)propyl, 2-(2-carbamoyl)propyl, 2-carbamoyl-2-methylpropyl, 4-carbamoylbutyl, 1-carbamoylbutyl, 1-(1-carbamoyl-2-methyl)butyl, 3-(4-carbamoyl-2-methyl)butyl.

Carboxy-C₁-C₄-alkoxy is, for example, carboxymethoxy, 2-carboxyethoxy, 2- or 3-carboxypropyloxy or 4-carboxybutyloxy, in particular carboxymethoxy.

Carboxy-C₁-C₄-alkyl is, for example, carboxymethyl, 2-carboxyethyl, 2- or 3-carboxypropyl, 2-carboxy-2-methylpropyl or 4-carboxybutyl, in particular carboxymethyl.

Cyano-C₁-C₄-alkoxy is, for example, cyanomethoxy, 2-cyanoethoxy, 2- or 3-cyanopropyloxy or 4-cyanobutyloxy, in particular cyanomethoxy.

Cyano-C₁-C₄-alkyl is, for example, cyanomethyl, 2-cyanoethyl, 2- or 3-cyanopropyl, 2-cyano-2-methylpropyl or 4-cyanobutyl, in particular cyanomethyl.

N,N-Di-C₁-C₄-alkylamino is, for example, dimethylamino, N-methyl-N-ethylamino, diethylamino, N-methyl-N-propylamino or N-butyl-N-methylamino.

N,N-Di-C₁-C₄-alkylamino-C₁-C₄-alkoxy is 2-dimethylaminoethoxy, 3-dimethylaminopropyloxy, 4-dimethylaminobutyloxy, 2-diethylaminoethoxy, 2-(N-methyl-N-ethylamino)ethoxy or 2-(N-butyl-N-methylamino)ethoxy.

N,N-Di-C₁-C₄-alkylamino-C₁-C₄-alkyl is, for example, 2-dimethylaminoethyl, 3-dimethylaminopropyl, 4-dimethylaminobutyl, 2-diethylaminoethyl, 2-(N-methyl-N-ethylamino)ethyl or 2-(N-butyl-N-methylamino)ethyl.

N,N-Di-C₁-C₄-alkylcarbamoyl-C₁-C₄-alkoxy is, for example, methyl- or dimethylcarbamoyl-C₁-C₄-alkoxy, such as N-methyl-, N-butyl- or N,N-dimethylcarbamoylmethoxy, 2-(N-methylcarbamoyl)ethoxy, 2-(N-butylcarbamoyl)ethoxy, 2-(N,N-dimethylcarbamoyl)ethoxy, 3-(N-methylcarbamoyl)propyloxy, 3-(N-butylcarbamoyl)propyloxy, 3-(N,N-dimethylcarbamoyl)propyloxy or 4-(N-methylcarbamoyl)butyloxy, 4-(N-butylcarbamoyl)butyloxy or 4-(N,N-dimethylcarbamoyl)butyloxy, in particular N-methyl-, N-butyl- or N,N-dimethylcarbamoylmethoxy.

N,N-Di-C₁-C₄-alkylcarbamoyl-C₁-C₄-alkyl is, for example, 2-dimethylcarbamoylethyl, 3-dimethylcarbamoylpropyl, 2-dimethylcarbamoylpropyl, 2-(dimethylcarbamoyl)-2-methylpropyl or 2-(1-dimethylcarbamoyl)butyl.

Optionally partially hydrogenated pyridyl- or N-oxidopyridyl-C₁-C₄-alkoxy is, for example, pyridyl- or N-oxidopyridylmethoxy, 2-pyridylethoxy, 2- or 3-pyridylpropyloxy or 4-pyridylbutyloxy, in particular 3- or 4-pyridylmethoxy.

Optionally partially hydrogenated pyridyl- or N-oxidopyridyl-C₁-C₄-alkyl is, for example, pyridyl- or N-oxidopyridylmethyl, 2-pyridylethyl, 2- or 3-pyridylpropyl or 4-pyridylbutyl, in particular 3- or 4-pyridylmethyl.

Halo-C₂-C₈-(hydroxy)alkoxy is, for example, halo-C₂-C₄-(hydroxy)alkoxy such as 3-halo- or 3-chloro-2-hydroxypropyloxy.

Hydroxy-C₂-C₈-alkoxy is, for example, hydroxy-C₂-C₄-alkoxy such as 2-hydroxybutyloxy, 3-hydroxypropyloxy or 4-hydroxybutyloxy.

Hydroxy-C₂-C₈-alkyl is, for example, hydroxy-C₂-C₄-alkyl such as 2-hydroxyethyl, 3-hydroxypropyl or 4-hydroxybutyl.

Morpholino-C₁-C₄-alkoxy may be N-oxidized and is, for example, 1-morpholinoethoxy, 3-morpholinopropyloxy or 1-(morpholino-2-methyl)propyloxy.

Morpholino-C₁-C₄-alkyl may be N-oxidized and is, for example, morpholinomethyl, 2-morpholinoethyl, 3-morpholinopropyl or 1- or 2-(4-morpholino)butyl.

C₁-C₈-Alkanoyl is in particular C₂-C₆-alkanoyl such as acetyl, propionyl, butyryl, isobutyryl or pivaloyl.

N-C₁-C₄-Alkanoylamino-C₁-C₄-alkyl is, for example, 2-acetaminoethyl.

C₁-C₈-Alkanol-C₂-C₄-alkoxy (oxo-C₂-C₈-alkoxy) bears the C₁-C₈-alkanoyl group in higher than the α-position and is, for example, 4-acetylbutoxy.

C₁-C₈-Alkanoyloxy-C₁-C₄-alkyl bears the C₁-C₈-alkanoyloxy group in higher than the α-position and is, for example, 4-acetoxybutyl.

C₁-C₈-Alkanesulphonyl-C₁-C₄-(hydroxy)alkoxy is, for example, 3-methanesulphonyl-2-hydroxypropyloxy.

C₁-C₈-Alkanesulphonyl-C₁-C₄-alkoxy is, for example, methanesulphonylmethoxy or 3-methanesulphonyl-2-hydroxypropyloxy.

C₁-C₈-Alkanesulphonylamino-C₁-C₄-alkoxy is, for example, ethanesulphonylaminomethoxy, 2-ethanesulphonylaminoethoxy, 3-ethanesulphonylaminopropyloxy or 3-(1,1 -dimethylethanesulphonylamino)propyloxy.

C₁-C₄-Alkanesulphonylamino-C₁-C₄-alkyl is, for example, ethanesulphonylaminomethyl, 2-ethanesulphonylaminoethyl, 3-ethanesulphonylaminopropyl or 3-(1,1-dimethylethanesulphonylamino)propyl.

C₁-C₈-Alkanesulphonyl-C₁-C₄-alkyl is, for example, ethanesulphonylmethyl, 2-ethanesulphonylethyl, 3-ethanesulphonylpropyl or 3-(1,1-dimethylethanesulphonyl)propyl.

C₂-C₈-Alkenyloxy is, for example, allyloxy.

C₂-C₈-Alkenyloxy-C₁-C₄-alkoxy is, for example, allyloxymethoxy.

C₂-C₈-Alkenyloxy-C₁-C₄-alkyl is, for example, allyloxymethyl.

C₁-C₈-Alkoxy is, for example, C₁-C₅-alkoxy such as methoxy, ethoxy, propyloxy, isopropyloxy, butyloxy, isobutyloxy, sec-butyloxy, tert-butyloxy or pentyloxy, but may also be a hexyloxy or heptyloxy group.

C₁-C₈-Alkoxycarbonyl is preferably C₂-C₅-alkoxycarbonyl such as methoxycarbonyl, ethoxycarbonyl, propyloxycarbonyl, isopropyloxycarbonyl, butyloxycarbonyl, isobutyloxycarbonyl, sec-butyloxycarbonyl or tert-butyloxycarbonyl.

C₁-C₈-Alkoxycarbonylamino-C₂-C₈-alkoxy is preferably C₂-C₅-alkoxycarbonylamino-C₂-C₈-alkoxy such as methoxycarbonylamino-C₂-C₈-alkoxy, ethoxycarbonylamino-C₂-C₈-alkoxy, propyloxycarbonylamino-C₂-C₈-alkoxy, isopropyloxycarbonylamino-C₂-C₈-alkoxy, butyloxycarbonylamino-C₂-C₈-alkoxy, isobutyloxycarbonylamino-C₂-C₈-alkoxy, sec-butyloxycarbonylamino-C₂-C₈-alkoxy or tert-butyloxycarbonylamino-C₂-C₈-alkoxy, in which C₂-C₈-alkoxy is, for example, ethoxy, propyloxy, butyloxy, pentyloxy or hexyloxy.

C₁-C₈-Alkoxycarbonylamino-C₂-C₈-alkyl is preferably C₂-C₅-alkoxycarbonylamino-C₂-C₈-alkyl such as methoxycarbonylamino-C₂-C₈-alkyl, ethoxycarbonylamino-C₂-C₈-alkyl, propyloxycarbonylamino-C₂-C₈-alkyl, isopropyloxycarbonylamino-C₂-C₈-alkyl, butyloxycarbonylamino-C₂-C₈-alkyl, isobutyloxycarbonylamino-C₂-C₈-alkyl, sec-butyloxycarbonylamino-C₂-C₈-alkyl or tert-butyloxycarbonylamino-C₂-C₈-alkyl, in which C₂-C₈-alkyl is, for example, ethyl, propyl, butyl, pentyl or hexyl.

C₁-C₄-Alkoxycarbonyl-C₁-C₄-alkoxy is, for example, methoxycarbonyl- or ethoxycarbonylmethoxy, 2-methoxycarbonyl- or 2-ethoxycarbonylethoxy, 2- or 3-methoxycarbonyl- or 2- or 3-ethoxycarbonylpropyloxy or 4-methoxycarbonyl- or 4-ethoxycarbonylbutyloxy, in particular methoxycarbonyl- or ethoxycarbonylmethoxy or 3-methoxycarbonyl- or 3-ethoxycarbonylpropyloxy.

C₁-C₄-Alkoxycarbonyl-C₁-C₄-alkyl is, for example, methoxycarbonyl- or ethoxycarbonylmethyl, 2-methoxycarbonyl- or 2-ethoxycarbonylethyl, 3-methoxycarbonyl- or 3-ethoxycarbonylpropyl or 4-ethoxycarbonylbutyl.

C₁-C₄-Alkoxy-C₂-C₄-alkenyl is, for example 4-methoxybut-2-enyl.

C₁-C₈-Alkoxy-C₁-C₈-alkoxy is, for example, 2-methoxy-, 2-ethoxy- or 2-propyloxyethoxy, 3-methoxy- or 3-ethoxypropyloxy or 4-methoxybutyloxy, in particular 3-methoxypropyloxy or 4-methoxybutyloxy.

C₁-C₄-Alkoxy-C₁-C₄-alkoxy-C₁-C₄-alkyl is, for example, 2-methoxy-, 2-ethoxy- or 2-propyloxyethoxymethyl, 2-(2-methoxy-, 2-ethoxy- or 2-propyloxyethoxy)ethyl, 3-(3-methoxy- or 3-ethoxypropyloxy)propyl or 4-(2-methoxybutyloxy)butyl, in particular 2-(3-methoxypropyloxy)ethyl or 2-(4-methoxybutyloxy)ethyl.

C₁-C₄-Alkoxy-C₁-C₄-alkyl is, for example, ethoxymethyl, propyloxymethyl, butyloxymethyl, 2-methoxy-, 2-ethoxy- or 2-propyloxyethyl, 3-methoxy- or 3-ethoxypropyl or 4-methoxybutyl, in particular 3-methoxypropyl or 4-methoxybutyl.

C₁-C₈-Alkyl may be straight-chain or branched and/or bridged and is, for example, methyl, ethyl, propyl, isopropyl, butyl, isobutyl, sec-butyl, tert-butyl, or a pentyl, hexyl or heptyl group.

C₂-C₈-Alkenyl may be straight-chain or branched and is, for example, vinyl, allyl, propenyl, isopropenyl, butenyl, isobutenyl or a pentenyl, hexenyl or heptenyl group.

C₂-C₆-Alkynyl may be straight-chain or branched and is, for example, ethynyl, 1-propnyl, 3-propnyl, 1-butnyl, 3-butnyl, 4-butnyl or a pentnyl or hexnyl group.

C₁-C₄-Alkylamino is, for example, methylamino, ethylamino, propylamino or butylamino.

C₁-C₄-Alkylamino-C₁-C₄-alkoxy is, for example, propylaminomethoxy, 2-methylamino-, 2-ethylamino-, 2-propylamino- or 2-butylaminoethoxy, 3-ethylamino- or 3-propylaminopropyloxy or 4-methylaminobutoxy.

C₁-C₄-Alkylamino-C₁-C₄-alkyl is, for example, propylaminomethyl, 2-methylamino-, 2-ethylamino-, 2-propylamino- or 2-butylaminoethyl, 3-ethylamino- or 3-propylaminopropyl or 4-methylaminobutyl.

N-C₁-C₈-Alkylcarbamoyl-C₁-C₄-alkoxy is, for example, methyl- or dimethylcarbamoyl-C₁-C₄-alkoxy, e.g. methylcarbamoylmethoxy, 2-methylcarbamoylethoxy or 3-methylcarbamoylpropyloxy.

C₀-C₆-Alkylcarbonylamino is, for example, carbonylamino, methylcarbonylamino, ethylcarbonylamino or propylcarbonylamino.

C₁-C₆-Alkylenedioxy is, for example, methylenedioxy or ethylenedioxy, but may also be 1,3- or 1,2-propylenedioxy.

C₁-C₄-Alkylthio-C₁-C₄-(hydroxy)alkoxy is, for example, 2-hydroxy-3-methylthiopropyloxy.

C₁-C₄-Alkylthio-C₁-C₄-alkoxy is, for example, methylthio-C₁-C₄-alkoxy, e.g. methylthiomethoxy, 2-methylthioethoxy or 3-methylthiopropyloxy.

C₁-C₄-Alkylthio-C₁-C₄-alkyl is, for example, methylthio-C₁-C₄-alkyl, e.g. methylthiomethyl, 2-methylthioethyl or 3-methylthiopropyl.

N'-C₂-C₈-Alkanoylpiperazino-C₁-C₄-alkyl is, for example, 4-acetylpiperazinomethyl.

N'-C₁-C₄-Alkylpiperazino-C₁-C₄-alkyl is 4-methylpiperazinomethyl.

Piperazino-C₁-C₄-alkyl is, for example, piperazinomethyl, 2-piperazinoethyl or 3-piperazinopropyl.

Piperidino-C₁-C₄-alkoxy is, for example, piperidinomethoxy, 2-piperidinoethoxy or 3-piperidinopropyloxy.

Piperidino-C₁-C₄-alkyl is, for example, piperidinomethyl, 2-piperidinoethyl or 3-piperidinopropyl.

Pyrrolidino-C₂-C₄-alkoxy is, for example, 2-pyrrolidinoethoxy or 3-pyrrolidinopropyloxy.

Pyrrolidino-C₁-C₄-alkyl is, for example, pyrrolidino-C₁-C₄-alkyl such as pyrrolidinomethyl, 2-pyrrolidinoethyl or 3-pyrrolidinopropyl.

S,S-Dioxothiomorpholino-C₁-C₄-alkyl is, for example, S,S-dioxothiomorpholinomethyl or 2-(S,S-dioxo)thiomorpholinoethyl.

S-Oxothiomorpholino-C₁-C₄-alkyl is, for example, S-oxothiomorpholinomethyl or 2-(S-oxo)thiomorpholinoethyl.

Thiazolyl-C₁-C₄-alkoxy is, for example, thiazolylmethoxy, 2-thiazolylethoxy or 3-thiazolylpropyloxy.

Thiomorpholino-C₁-C₄-alkyl or S,S-dioxothiomorpholino-C₁-C₄-alkyl is, for example, thiomorpholino-C₁-C₄-alkyl such as -methyl or -ethyl, or S,S-dioxothiomorpholino-C₁-C₄-alkyl such as -methyl or -ethyl.

Carbamoyl or N-mono- or N,N-di-C₁-C₈-alkylated carbamoyl is, for example, carbamoyl, methylcarbamoyl, ethylcarbamoyl, N,N-dimethylcarbamoyl, N,N-diethylcarbamoyl or propylcarbamoyl.

Carboxyl or esterified carboxyl is, for example, carboxyl esterified with C₀-C₆-alkyl, such as carboxyl or C₁-C₆-alkoxycarbonyl.

Depending on the presence of asymmetric carbon atoms, the inventive compounds may be present in the form of isomer mixtures, especially as racemates, or in the form of pure isomers, especially of optical antipodes. The invention encompasses all of these forms. Diastereomer mixtures, diastereomeric racemates or mixtures of diastereomeric racemates may be separated by customary methods, for example by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulpho group, and are for example their salts with suitable bases, such as non-toxic metal salts derived from metals of group Ia, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as unsubstituted or hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N.N-di-lower-alkyl-N-(hydroxylower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of the formula (I) also include those compounds in which one or more atoms are replaced by their stable, non-radioactive isotopes; for example, a hydrogen atom by deuterium.

The above and hereafter specified compound groups are not to be regarded as closed, but rather it is possible in a sensible manner, for example in order to replace general by more specific definitions, to exchange parts of these compound groups with one another or for the definitions given or to omit them.

The invention preferably relates to compounds of the formula (I), in which R₁ is aryl which is unsubstituted or mono- or polysubstituted by C₁-C₈-alkyl, cyano, hydroxyl, amino, C₁-C₆-alkylamino, N,N-di-C₁-C₆-alkylamino, C₂-C₆-alkenyl, C₂-C₆-alkynyl, C₀-C₆-alkylcarbonylamino, C₁-C₆-alkoxycarbonylamino, halogen, trifluoromethyl, C₁-C₈-alkoxy, carbamoyl or N-mono- or N,N-di-C₁-C₈-alkylated carbamoyl, carboxyl or esterified carboxyl, aryl or heterocyclyl,
R₂ is C₁-C₈-alkyl;
R₃ is hydrogen;
R₄ is hydrogen;
R₅ are each independently hydrogen or C₁-C₈-alkyl;
R₆ is hydrogen;
R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy;
and their pharmaceutically usable salts.

Preference is likewise given to compounds of the formula (I) in which R₁ is aryl substituted by C₁-C₆-alkylenedioxy;
R₂ is C₁-C₈-alkyl;
R₃ is hydrogen;
R₄ is hydrogen;
R₅ are each independently hydrogen or C₁-C₈-alkyl;
R₆ is hydrogen;
R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy;
and their pharmaceutically usable salts.

Preference is likewise given to compounds of the formula (I) in which R₂ is C₁-C₈-alkyl;
R₃ is hydrogen;
R₄ is hydrogen;
R₅ are each independently hydrogen or C₁-C₈-alkyl;
R₆ is hydrogen; and
R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy.

Preference is likewise given to compounds of the formula (I) in which R₂ is C₁-C₈-alkyl. Preference is likewise given to compounds of the formula (I) in which R₃ is hydrogen. Preference is likewise given to compounds of the formula (I) in which R₄ is hydrogen. Preference is likewise given to compounds of the formula (I) in which R₅ are each independently hydrogen or C₁-C₈-alkyl, particular preference being given to one R₅ radical being hydrogen and one R₅ radical being C₁-C₈-alkyl. Preference is likewise given to compounds of the formula (I) in which R₆ is hydrogen. Preference is likewise given to compounds of the formula (I) in which R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy.

The invention further preferably relates to compounds of the formula 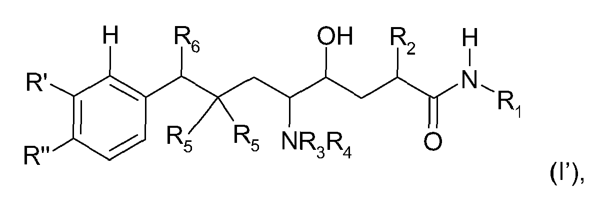 in which
R₁, R2, R₃, R₄, R₅ and R₆ are each as defined for the compounds of the formula (I) and
R' and R" are each independently as defined for R for the compounds of the formula (I) and are preferably hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy or C₁-C₄-alkoxy-C₁-C₄-alkoxy.

The compounds of the formula (I) may also be prepared in optically pure form. The separation into antipodes may be effected by methods known per se, either preferably at a synthetically early stage by salt formation with an optically active acid, for example (+)- or (-)-mandelic acid and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the compound present, the pure diastereomeric salts and derivatives may be analysed with common spectroscopic methods, of which X-ray spectroscopy on single crystals constitutes a particularly suitable method.

Particular preference is given in each case to those compounds of the formula (I) in which at least one asymmetric carbon atom, for example one, two, three or preferably all four asymmetric carbon atoms, of the main chain have the stereochemistry shown in formula (IA) (each "S"), the substituents each being as defined above, and their pharmaceutically usable salts:

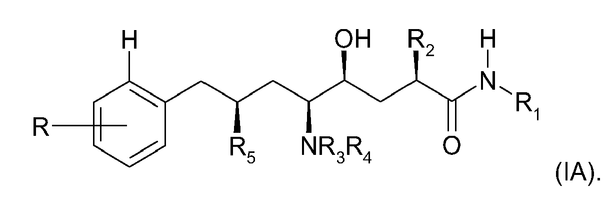

The compounds of the formula (I), (I') or formula (IA) may be prepared analogously to the literature preparation processes (see WO 2002/008172 and WO 2002/002508 or literature cited there) (scheme).

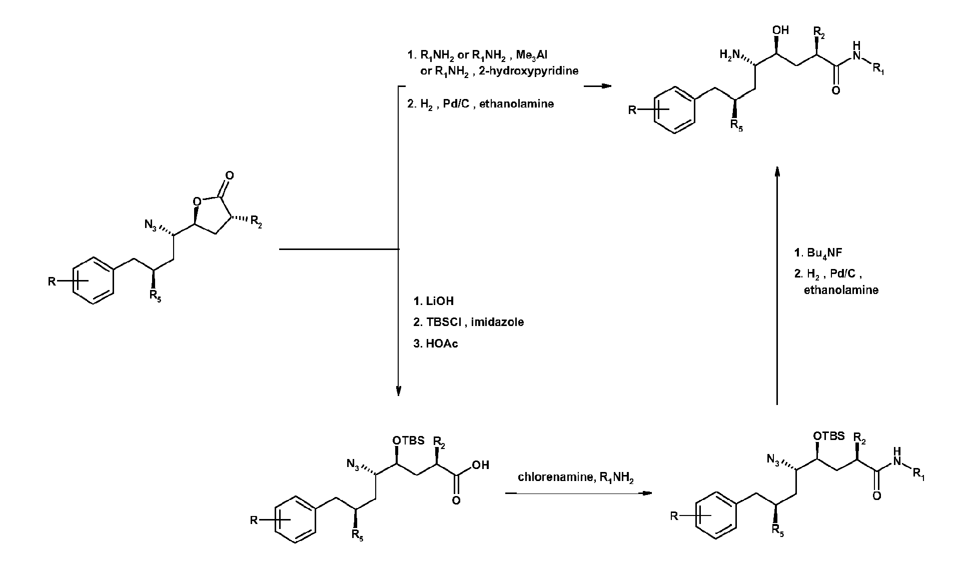

Details of the specific preparation variants can be taken from the examples.

The compounds of formula (I), (I') and (IA), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1% BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems eg the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formulae (I') or (IA) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) or of preferred formulae (I') or (IA) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I), (I') and (IA), respectively, and their pharmaceutically useful salts for the prevention, delay of progression or the treatment of Alzheimer Disease, malaria or HIV infection.

Subject of the present invention is also the use of the compounds of formula (I), (I') and (IA), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formulae (I') and (IA) or a pharmaceutically usable salt thereof is applied.

Subject of the present invention is also a pharmaceutical preparation that contains for the inhibition of beta-secretase, cathepsin D, plasmepsin and/or HIV-protease a compound of the general formula (I), or preferred of formulae (I') and (IA) or a pharmaceutically usable salt thereof as well as commonly used ingredients.

Subject of the present invention is also a pharmaceutical preparation for the prevention, delay of progression or treatment of Alzheimer Disease, malaria and HIV infection that contains a compound of the general formula (I), or preferred of formula (I') and (IA) or a pharmaceutically usable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is determined in the solvent system A. The ratio of solvents relative to one another is always reported in parts by volume. Chemical names for end products and intermediates were generated with the aid of the program AutoNom 2000 (automatic nomenclature).

HPLC gradients on Hypersil BDS C-18 (5 µm); column: 4 ×125 mm
I 90% water*/10% acetonitrile* to 0% water*/100% acetonitrile* in 5 minutes + 2.5 minutes (1.5 ml/min)
II 95% water*/5% acetonitrile* to 0% water*/100% acetonitrile* in 40 minutes (0.8 ml/min)
   * containing 0.1 % trifluoroacetic acid

The following abbreviations are used:
- Rf: ratio of distance travelled by a substance to separation of the eluent front from the start point in thin-layer chromatography
- Rt: retention time of a substance in HPLC (in minutes)
- m.p.: melting point (temperature)

### General method A: (azide reduction)

A solution of 1 mmol of "azide derivative" in 10-20 ml of ethanol and ethanolamine (1 equiv.) is hydrogenated in the presence of 200-400 mg of 10% Pd/C (moist) at 0°C over 1-3 hours. The reaction mixture is clarified by filtration and the catalyst is washed with ethanol. The filtrate is concentrated by evaporation. The title compound is obtained from the residue by means of flash chromatography (SiO₂ 60F).

### General method B: (Lactone amidation I)

A mixture of 1 mmol of "lactone", "amine" (10-30 equiv.) and 2-hydroxypyridine (1 equiv.) is stirred at 65°C over 2-24 hours. The reaction mixture is cooled to room temperature, concentrated by evaporation, admixed ith 1 M aqueous sodium hydrogencarbonate solution and extracted with tert-butyl methyl ether (2x). The combined organic phases are washed with water and brine, dried over sodium sulphate and concentrated. The title compound is obtained from the residue by means of flash chromatography (SiO₂ 60F).

### General method C: (Lactone amidation II)

A solution of 1.1 mmol of trimethylaluminium solution (2 M in heptane) at -78°C is admixed with a solution of 1.2 mmol of "amine" in 1-2 ml of toluene. The reaction mixture is warmed to room temperature, stirred for a further 30-60 minutes and subsequently concentrated by evaporation. The residue is admixed with a solution of 1 mmol of "lactone" in 2 ml of toluene and stirred at 80°C for 2-4 hours. The reaction mixture is cooled to room temperature, admixed with 10 ml of 1 N HCl and then stirred for a further 30 minutes. The reaction mixture is diluted with brine and extracted with toluene (2x) - the combined organic phases are dried over sodium sulphate and concentrated by evaporation. The title compound is obtained from the residue by means of flash chromatography (SiO₂ 60F).

### Example 1:

### N-Phenyl-5(S)-amino-4(S)-hydroxy-2(S)-isopropyl-7(S)-f4-methoxy-3-(3-methoxypropoxy)benzyl]-8-methylnonanamide

Analogously to Method A, 0.132 g of N-phenyl-5(S)-azido-4(S)-hydroxy-2(S)-isopropyl-7(S)-[4-methoxy-3-(3-methoxypropoxy)benzyl]-8-methylnonanamide is used to prepare the title compound as a colourless foam. Rf = 0.45 (200:20:1 dichloromethane-methanol-25% conc. ammonia); Rt = 4.36 minutes (gradient I).

The starting material is prepared as follows:

### a) N-Phenyl-5(S)-azido-4(S)-hydroxy-2(S)-isopropyl-7(S)-[4-methoxy-3-(3-methoxypropoxy)benzyl]-8-methylnonanamide

Analogously to Method C, 0.466 g of 5(S)-{1(S)-azido-3(S)-[4-methoxy-3-(3-methoxypropoxy)benzyl]-4-methylpentyl}-3(S)-isopropyldihydrofuran-2-one [324763-46-4] and 0.11 ml of aniline are reacted. The title compound is obtained as a colourless oil.
Rf = 0.53 (2:1 EtOAc-heptane); Rt = 5.41 minutes (gradient I).

The process described in Example 1 is used analogously to prepare the following compounds:

### Examples

2 N-(2-Fluorophenyl)-5(S)-amino-4(S)-hydroxy-2(S)-isopropyl-7(S)-[4-methoxy-3-(3-methoxypropoxy)benzyl]-8-methylnonanamide
3 N-(3-Fluorophenyl)-5(S)-amino-4(S)-hydroxy-2(S)-isopropyl-7(S)-[4-methoxy-3-(3-methoxypropoxy)benzyl]-8-methylnonanamide
4 N-(4-Fluorophenvi)-5(S)-amino-4(S)-hydroxy-2(S)-isopropyl-7(S)-[4-methoxy-3-(3-methoxypropoxy)benzyl]-8-methylnonanamide
5 N-(4-Methoxyphenyl)-5(S)-amino-4(S)-hydroxy-2(S)-isopropyl-7(S)-[4-methoxy-3-(3-methoxypropoxy)benzyl]-8-methylnonanamide
6 N-(2-Methoxyphenyl)-5(S)-amino-4(S)-hydroxy-2(S)-isopropyl-7(S)-[4-methoxy-3-(3-methoxypropoxy)benzyl]-8-methylnonanamide
7 N-(3-Methoxyphenyl)-5(S)-amino-4(S)-hydroxy-2(S)-isopropyl-7(S)-[4-methoxy-3-(3-methoxypropoxy)benzyl]-8-methylnonanamide
8 N-(2-Cyanophenyl)-5(S)-amino-4(S)-hydroxy-2(S)-isopropyl-7(S)-[4-methoxy-3-(3-methoxypropoxy)benzyl]-8-methylnonanamide
9 N-(3-Cyanophenyl)-5(S)-amino-4(S)-hydroxy-2(S)-isopropyl-7(S)-f4-methoxy-3-(3-methoxypropoxy)benzyll-8-methylnonanamide
10 N-(4-Cyanophenvi)-5(S)-amino-4(S)-hydroxy-2(S)-isopropyl-7(S)-[4-methoxy-3-(3-methoxypropoxy)benzyll-8-methylnonanamide
11 N-(Benzo[1,3]dioxol-5-yl)-5(S)-amino-4(S)-hydroxy-2(S)-isopropyl-7(S)-[4-methoxy-3-(3-methoxypropoxy)benzyll-8-methylnonanamide
12 N-(Benzo[1,3]dioxol-4-yl)-5(S)-amino-4(S)-hydroxy-2(S)-isopropyl-7(S)-[4-methoxy-3-(3-methoxypropoxy)benzyl]-8-methylnonanamide
13 N-(2,3-Dihydrobenzo[1,4]dioxin-6-yi)-5(S)-amino-4(S)-hydroxy-2(S)-isopropyl-7(S)-[4-methoxy-3-(3-methoxypropoxy)benzyl]-8-methylnonanamide
14 N-(2,3-Dihydrobenzo[1,4]dioxin-5-yl)-5(S)-amino-4(S)-hydroxy-2(S)-isopropyl-7(S)-[4-methoxy-3-(3-methoxypropoxy)benzyl]-8-methylnonanamide

## Claims

1. Use of a compound of formula (I) 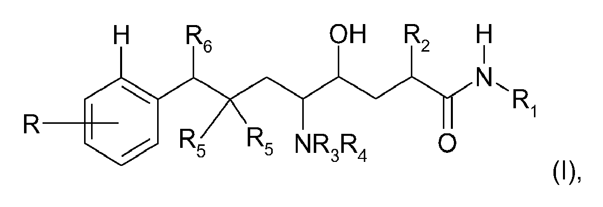 in which
R₁ is unsubstitited or substituted aryl;
R₂ is C₁-C₈-alkyl, C₂-C₈-alkenyl or C₃-C₈-cycloalkyl or is phenyl- or naphthyl-C₁-C₄-alkyl, each of which is unsubstituted or mono-, di- or trisubstituted by C₁-C₄-alkyl, C₁-C₄-alkoxy, hydroxyl, C₁-C₄-alkylamino, N,N-di-C₁-C₄-alkylamino, halogen and/or trifluoromethyl;
R₃ is hydrogen, C₁-C₄-alkyl or C₁-C₈-alkanoyl;
R₄ is hydrogen, C₁-C₄-alkyl or C₁-C₈-alkanoyl;
R₅ are each independently hydrogen, C₁-C₈-alkyl or, together with the carbon atom to which they are bonded, a C₃-C₈-cycloalkylidene radical;
R₆ is hydrogen or hydroxyl;
R are each independently 1-4 radicals selected from:
hydrogen, halogen, C₁-C₈-alkyl, 3- to 8-membered cycloalkyl, polyhalo-C₁-C₄-alkyl, polyhalo- C₁-C₄-alkoxy, C₁-C₄-alkoxy-C₁-C₄-alkyl, C₁-C₄-alkoxy-C₁-C₄-alkoxy-C₁-C₄-alkyl, 3- to 8-membered cycloalkoxy-C₁-C₄-alkyl, hydroxyl, C₁-C₈-alkanoyloxy-C₁-C₄-alkyl, hydroxy-C₂-C₈-alkyl, C₁-C₄-alkylthio-C₁-C₄-alkyl, C₁-C₈-alkanesulphonyl-C₁-C₄-alkyl, thiazolylthio-C₁-C₄-alkyl, thiazolinylthio-C₁-C₄-alkyl, imidazolylthio-C₁-C₄-alkyl, optionally N-oxidized pyridylthio-C₁-C₄-alkyl, pyrimidinylthio-C₁-C₄-alkyl, optionally partially hydrogenated pyridyl- or N-oxidopyridyl-C₁-C₄-alkyl, C₁-C₄-alkanesulphonylamino-C₁-C₄-alkyl, trifluoro-C₁-C₈-alkanesulphonylamino-C₁-C₄-alkyl, pyrrolidino-C₁-C₄-alkyl, piperidino-C₁-C₄-alkyl, piperazino-C₁-C₄-alkyl, N'-C₁-C₄-alkylpiperazino-C₁-C₄-alkyl, N'-C₂-C₈-alkanoylpiperazino-C₁-C₄-alkyl, morpholino-C₁-C₄-alkyl, thiomorpholino-C₁-C₄-alkyl, S-oxothiomorpholino-C₁-C₄-alkyl, S,S-dioxothio-morpholino-C₁-C₄-alkyl, cyano-C₁-C₄-alkyl, carboxy-C₁-C₄-alkyl, C₁-C₄-alkoxycarbonyl-C₁-C₄-alkyl, carbamoyl-C₁-C₈-alkyl, N-mono- or N,N-di-C₁-C₄-alkylcarbamoyl-C₁-C₄-alkyl, unsubstituted phenyl or by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted phenyl, unsubstituted naphthyl or by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted naphthyl, hydroxy-C₂-C₈-alkoxy, halo-C₂-C₈-(hydroxy)alkoxy, C₁-C₈-alkanesulphonyl-C₁-C₄-(hydroxy)alkoxy, amino-C₁-C₄-alkyl, C₁-C₄-alkylamino-C₁-C₄-alkyl, N,N-di-C₁-C₄-alkylamino-C₁-C₄-alkyl, N-C₁-C₄-alkanoylamino-C₁-C₄-alkyl, C₁-C₈-alkoxycarbonylamino-C₂-C₈-alkyl, amino-C₁-C₄-alkoxy, C₁-C₄-alkylamino-C₁-C₄-alkoxy, N,N-di-C₁-C₄-alkylamino-C₁-C₄-alkoxy, C₁-C₈-alkanoylamino-C₁-C₄-alkoxy, C₁-C₈-alkoxycarbonylamino-C₂-C₈-alkoxy, C₁-C₈-alkanoyl-C₂-C₄-alkoxy which bears the alkanoyl group in higher than the α-position, C₁-C₈-alkoxy, 3- to 8-membered cycloalkoxy, C₂-C₈-alkenyloxy, 3- to 8-membered cycloalkoxy-C₁-C₄-alkoxy, C₁-C₄-alkoxy-C₁-C₄-alkoxy, C₁-C₄-alkoxy-C₂-C₄-alkenyl, C₂-C₈-alkenyloxy-C₁-C₄-alkoxy, C₁-C₄-alkoxy-C₂-C₄-alkenyloxy, C₂-C₈-alkenyloxy-C₁-C₄-alkyl, C₁-C₄-alkylthio-C₁-C₄-alkoxy, C₁-C₈-alkanesulphonyl-C₁-C₄-alkoxy, C₁-C₄-alkylthio-C₁-C₄-(hydroxy)alkoxy, optionally by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted phenyl-C₁-C₄-alkoxy, optionally by C₁-C₄-alkyl, C₁-C₄-alkoxy, hydroxyl, C₁-C₄-alkylamino, di-C₁-C₄-alkylamino, halogen and/or trifluoromethyl mono-, di- or trisubstituted naphthyl-C₁-C₄-alkoxy, each of which is unsubstituted or mono-, di- or trisubstituted by C₁-C₄-alkyl, C₁-C₄-alkoxy, hydroxyl, C₁-C₄-alkylamino, di-C₁-C₄-alkylamino, halogen and/or trifluoromethyl, polyhalo-C₁-C₄-alkoxy, optionally partially hydrogenated pyridyl- or N-oxidopyridyl-C₁-C₄-alkoxy, thiazolyl-C₁-C₄-alkoxy, optionally N-oxidized morpholino-C₁-C₄-alkoxy, thiazolylthio-C₁-C₄-alkoxy, thiazolinylthio-C₁-C₄-alkoxy, imidazolylthio-C₁-C₄-alkoxy, optionally N-oxidized pyridylthio-C₁-C₄-alkoxy, pyrimidinylthio-C₁-C₄-alkoxy, C₁-C₈-alkanesulphonylamino-C₁-C₄-alkoxy, trifluoro-C₁-C₈-alkanesulphonyl-C₁-C₄-alkoxy, pyrrolidino-C₁-C₄-alkoxy, piperidino-C₁-C₄-alkoxy, cyano-C₁-C₄-alkoxy, carboxy-C₁-C₄-alkoxy, C₁-C₄-alkoxycarbonyl-C₁-C₄-alkoxy, carbamoyl-C₁-C₄-alkoxy, N-C₁-C₈-alkylcarbamoyl-C₁-C₄-alkoxy, N-mono- or N,N-di-C₁-C₄-alkylcarbamoyl-C₁-C₄-alkoxy, carboxy-C₁-C₄-alkyl, C₁-C₄-alkoxycarbonyl-C₁-C₄-alkyl, carbamoyl-C₁-C₈-alkyl, N-mono- or N,N-di-C₁-C₄-alkylcarbamoyl-C₁-C₄-alkyl, carboxy-C₁-C₄-alkoxy, C₁-C₄-alkoxycarbonyl-C₁-C₄-alkoxy, carbamoyl-C₁-C₈-alkoxy, C₁-C₄-alkylamino or N,N-di-C₁-C₄-alkylamino,
or pharmaceutically usable salt or prodrug thereof, or where one or more atoms are replaced by their stable, non-radioactive isotopes,
for the manufacture of a medicament for the inhibition of beta-secretase, cathepsin D, plasmepsin II and/or HIV-protease.

2. Use according to claim 1, in which
R₁ is unsubstituted aryl or aryl mono- or polysubstituted by C₁-C₈-alkyl, cyano, hydroxyl, amino, C₁-C₆-alkylamino, N,N-di-C₁-C₆-alkylamino, C₂-C₆-alkenyl, C₂-C₆-alkynyl, C₀-C₆-alkylcarbonylamino, C₁-C₆-alkoxycarbonylamino, halogen, trifluoromethyl, C₁-C₈-alkoxy, carbamoyl or N-mono- or N,N-di-C₁-C₈-alkylated carbamoyl, carboxyl or esterified carboxyl, aryl or heterocyclyl.

3. Use according to claim 1, in which
R₁ is aryl substituted by C₁-C₆-alkylenedioxy.

4. Use according to one of claims 1 to 3 of a compound of formula (1') 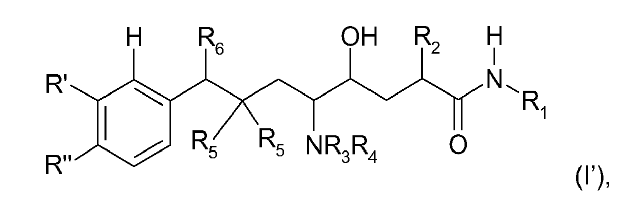 in which
R₁, R2, R₃, R₄, R₅ and R₆ are each as defined for the compound of the formula (I) and R' and R" are each independently as defined for R for the compound of the formula (I).

5. Use according to one of claims 1 to 4 of a compound of formula (IA) 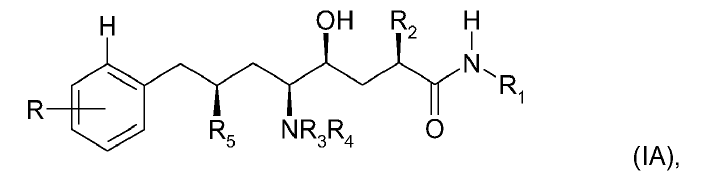 in which R, R₁, R₂, R₃, R₄ and R₅ are each as defined for the compound of the formula (1).

6. Use according to one of claims 1 to 5, in which for formulae (I) and (IA)
R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy, and for formula (I') R' and R" are each independently as defined for R.

7. Use according to one of claims 1 to 6, in which for formulae (I), (I') and (IA)
R₁ is unsubstituted or substituted aryl,
R₂ is C₁-C₈-alkyl;
R₃ is hydrogen;
R₄ is hydrogen;
R₅ are each independently hydrogen or C₁-C₈-alkyl;
R₆ is hydrogen;
for formulae (I) and (IA) R are each independently 1-4 radicals selected from:
hydrogen, halogen, C₁-C₄-alkyl, C₁-C₄-alkoxy-C₁-C₄-alkyl, C₁-C₄-alkoxy-C₁-C₄-alkoxy-C₁-C₄-alkyl, C₁-C₈-alkoxy and C₁-C₄-alkoxy-C₁-C₄-alkoxy, and for formula (I') R' and R" are each independently radicals as defined for R.

8. Use of a compound of the general formula (I), (IA) or (I'), or a pharmaceutically usable salt thereof, according to any one of claims 1 to 7 for the preparation of a medication for the prevention, delay of progression or treatment of Alzheimer Disease, malaria or HIV infection.

9. Pharmaceutical preparation for the prevention, delay of progression or treatment of Alzheimer disease, malaria or HIV infection containing a compound of the general formula (I), (IA) or (I'), or a pharmaceutically usable salt thereof, according to any one of claims 1 to 7 as well as commonly used ingredients.
